# EUROPEAN PATENT APPLICATION

(11) **EP 3 912 970 A1**
(43) Date of publication of application: **24.11.2021**
(21) Application number: 20740924.4
(22) Date of filing: 14.01.2020
(51) Int. Cl.: C07C 327/32

(54) **METHOD FOR PREPARING S-(-)-BENZOYLMERCAPTO-2-METHYLPROPANOIC ACID COMPOUND**

(30) Priority: 14.01.2019 CN 201910031282
(71) Applicant: Zhejiang Huahai Pharmaceutical Co., Ltd., Taizhou, Zhejiang 317024 (CN); Zhejiang Huahai Licheng Pharmaceutical Co., Ltd., Taizhou, Zhejiang 317024 (CN); Zhejiang Huahai Tiancheng Pharmaceutical Co., Ltd, Taizhou, Zhejiang 318000 (CN)
(72) Inventor: ZHANG, Jian, Zhejiang 317024 (CN); HUANG, Wenfeng, Zhejiang 317024 (CN); JIN, Lei, Zhejiang 317024 (CN); HAN, Zheng, Zhejiang 317024 (CN); ZHENG, Guoxi, Zhejiang 317024 (CN)
(74) Representative: Pfenning, Meinig & Partner mbB
(86) International application number: PCT/CN2020/071927
(87) International publication number: WO 2020/147704

(57) **Abstract**

Disclosed is a method for synthesizing a S-(-)-benzoylmercapto-2-methylpropanoic acid compound, comprising hydrolyzing S-(-)-3-acetylmercapto-2-methylpropanoic acid represented by formula (II) as a raw material in a reaction solvent to obtain S-(-)-3-mercapto-2-methylpropanoic acid represented by formula (III), followed by condensing with benzoyl chloride to obtain S-(-)-benzoylmercapto-2-methylpropanoic acid represented by formula (I). The method of the present application is simple to implement, and the raw and auxiliary materials are cheap and readily available. The reaction conditions are mild, the yield is high, and the quality is high.

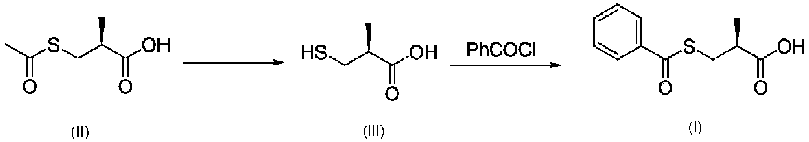

## Description

This application claims priority of China Patent Application No. 201910031282.4, filed with the Chinese Patent Office on January 14, 2019, titled "Method for Preparing S-(-)-Benzoylmercapto-2-Methylpropanoic Acid Compound" , which is hereby incorporated by reference in its entirety.

### FIELD OF THE INVENTION

The present application is directed to a method for synthesizing S-(-)-benzoylmercapto-2-methylpropanoic acid, an intermediate of zofenopril.

### BACKGROUND OF THE INVENTION

Zofenopril is a sulfhydryl-containing angiotensin converting enzyme inhibitor, which has good therapeutic effects on the treatment of hypertension, heart failure and acute myocardial infarction, etc. Its structural formula is as follows:

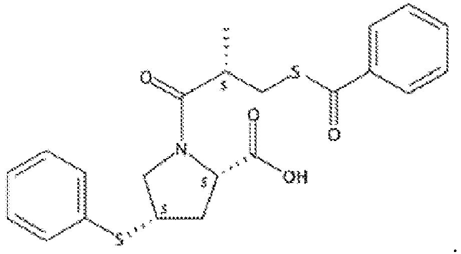

As an important intermediate of zofenopril, S-(-)-benzoylmercapto-2-methylpropanoic acid has its structural formula represented by formula I:

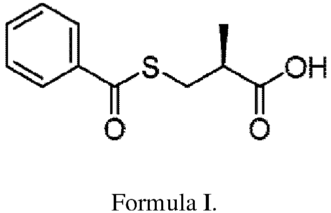

Currently there are two methods for the preparation of S-(-)-benzoylmercapto-2-methylpropanoic acid: 1. carry out an addition reaction of thiobenzoic acid with 2-methacrylic acid followed by a resolution to obtain S-(-)-benzoylmercapto-2-methylpropanoic acid, wherein the raw material thiobenzoic acid is expensive, and many side reactions occur during the preparation process, which leads to high environmental pollution; 2. carry out an addition reaction of thioacetic acid with 2-methacrylic acid followed by hydrolysis, and then condense with benzoyl chloride, and finally obtain S-(-)-benzoylmercapto-2-methylpropanoic acid through resolution, wherein many side reactions occur during a hydrolysis process, which leads to a low overall yield.

In view of the above problems, it is quite necessary to develop a synthetic route to synthesize S-(-)-benzoylmercapto-2-methylpropanoic acid with simple operation, mild conditions, few side reactions and a high overall yield.

### SUMMARY OF THE INVENTION

The present application provides a novel method for synthesizing S-(-)-benzoylmercapto-2-methylpropanoic acid, comprising the following steps:
(a) hydrolyzing S-(-)-3-acetylmercapto-2-methylpropanoic acid represented by formula II as a raw material in a reaction solvent to obtain S-(-)-3-mercapto-2-methylpropanoic acid represented by formula III;
(b) condensing S-(-)-3-mercapto-2-methylpropanoic acid represented by formula III with benzoyl chloride to obtain S-(-)-benzoylmercapto-2-methylpropanoic acid represented by formula I.

The reaction formula of the method of this application is as follows:

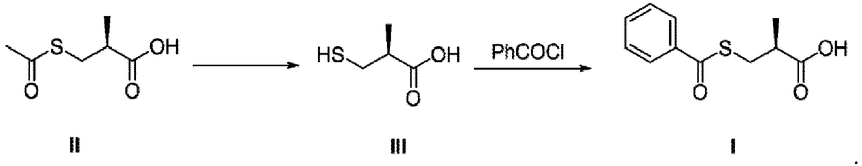

In some embodiments of the application, the hydrolysis in step (a) is preferably carried out in the presence of both alkali and a metal. The alkali is preferably inorganic alkali, more preferably sodium hydroxide or potassium hydroxide. The metal is selected from one of the group consisting of copper powder, iron powder, zinc powder, aluminum powder, nickel powder, or cobalt powder, or any mixture thereof, more preferably zinc powder. The mass amount of the metal is 0.5%-10%, preferably 2% of the mass of S-(-)-3-acetylmercapto-2-methylpropanoic acid represented by formula II.

In some embodiments of the application, the reaction solvent in step (a) is chosen from an organic solvent, water, or the mixture thereof, wherein the organic solvent is selected from one of the group consisting of C₁-C₄ alcohols, C₆-C₈ alkanes, toluene, ethyl acetate, or any mixture thereof. More preferably, the reaction solvent is water.

In some embodiments of the application, the ratio of the volume of the reaction solvent to the mass of S-(-)-3-acetylmercapto-2-methylpropanoic acid represented by formula II in step (a) is generally 1-10 mL/g, preferably 2 mL/g.

The temperature of the hydrolysis in step (a) in not limited in this application, and may be any temperatures sufficient to carry out the hydrolysis of S-(-)-3-acetylmercapto-2-methylpropanoic acid, which, for example, can be -10°C to 20°C, -5°C to 5°C, or approximately 0°C.

In some embodiments of the application, the molar ratio of the benzoyl chloride to S-(-)-3-acetylmercapto-2-methylpropanoic acid represented by formula II in step (b) is generally 100%-120%, preferably 120%.

The temperature of the condensation in step (b) is not limited in this application, and may be any temperatures sufficient to carry out the condensation of S-(-)-3-mercapto-2-methylpropanoic acid with the benzoyl chloride, which, for example, can be 10°C-30°C, or approximately 15°C.

In some embodiments of the application, the product represented by formula I can be crystallized with an appropriate solvent after the reaction in step (b) completed and subsequently a post-treatment. The solvent for crystallization is selected from ethyl acetate, n-heptane, or a combination thereof. The ratio of the volume of the solvent for crystallization to the mass of S-(-)-3-acetylmercapto-2-methylpropanoic acid represented by formula II is generally 1-20 mL/g, preferably 10 mL/g.

The synthesis method for synthesizing S-(-)-benzoylmercapto-2-methylpropanoic acid provided in the present application has the advantages of simple operation, cheap raw materials, mild reaction conditions, a high yield, high quality and few side reactions. Furthermore, the method in this application can permit the reaction in water.

### DETAILED DESCRIPTION OF THE INVENTION

For the sake of clarity of the purpose, technical solutions and advantages of the present application, the application is further described in detail in combination with specific examples. Obviously, the specific examples are only a part of the examples of the present application, rather than all the examples. Based on the examples in this application, all other examples obtained by those of ordinary skill in the art without creative work shall fall within the protection scope of this application.

All raw materials used in the Examples are commercially available.

### Example 1

Under the protection of nitrogen, 21.5 g sodium hydroxide, 90 mL potable water and 0.4 g zinc powder were added into a three-necked flask sequentially, and then cooled to 0 °C followed by adding dropwise 20 g S-(-)-3-acetylmercapto-2-methylpropionic acid slowly. The reaction was held for 3h. After the reaction was completed, pH was adjusted to 9.0 with concentrated hydrochloric acid. 20 g benzoyl chloride and 36 mL 4 mol/L of sodium hydroxide aqueous solution were added dropwise simultaneously when the temperature of the feed liquid was controlled at 15°C. After the reaction was completed, the pH was adjusted to 2.0 with concentrated hydrochloric acid, and 80 mL ethyl acetate was added for extraction. After layering, 120 mL n-heptane was added to a layer of ethyl acetate, and the mixture was stirred and crystallized for 4 hours. The crystal thus obtained was filtered and dried. 19.4 g S-(-)-benzoylmercapto-2-methylpropanoic acid was obtained. Yield: 71.9%. HPLC purity: 99.90%. Optical purity: 99.2%.

### Example 2

Under the protection of nitrogen, 21.5 g sodium hydroxide, 90 mL potable water and 0.2 g aluminum powder were added into a three-necked flask sequentially, and then cooled to 0°C followed by adding dropwise 20 g S-(-)-3-acetylmercapto-2-methylpropionic acid slowly. The reaction was held for 3h. After the reaction was completed, pH was adjusted to 9.0 with concentrated hydrochloric acid. 22 g benzoyl chloride and 36 mL 4 mol/L of sodium hydroxide aqueous solution were added dropwise simultaneously when the temperature of the feed liquid was controlled at 15°C. After the reaction was completed, the pH was adjusted to 2.0 with concentrated hydrochloric acid, and 80 mL ethyl acetate was added for extraction. After layering, 120 mL n-heptane was added to a layer of ethyl acetate, and the mixture was stirred and crystallized for 4 hours. The crystal thus obtained was filtered and dried. 19.2 g S-(-)-benzoylmercapto-2-methylpropanoic acid was obtained. Yield: 66.7%. HPLC purity: 99.0%. Optical purity: 99.0%.

Hence it can be seen that the method of the present application for synthesizing S-(-)-benzoylmercapto-2-methylpropanoic acid is simple to carry out, and the raw materials and auxiliary materials are cheap and readily available. The reaction conditions are mild. Furthermore, with the method of the present application, a yield of product can be more than 65%, or even more than 70%. A purity of the product can be as high as 99.0%, and the optical purity can be more than 99%.

Examples mentioned above are only the preferred examples of the present application and are not intended to limit this application. Any modification, equivalent replacement, improvement, etc. made within the spirit and principle of this application shall be included within the protection scope of the present application.

## Claims

1. A method for synthesizing S-(-)-benzoylmercapto-2-methylpropanoic acid represented by formula I, comprising:
(a) hydrolyzing S-(-)-3-acetylmercapto-2-methylpropanoic acid represented by formula II as a raw material in a reaction solvent to obtain S-(-)-3-mercapto-2-methylpropanoic acid represented by formula III; and
(b) condensing S-(-)-3-mercapto-2-methylpropanoic acid represented by formula III with benzoyl chloride to obtain S-(-)-benzoylmercapto-2-methylpropanoic acid represented by formula I;
wherein the reaction scheme is as follows:

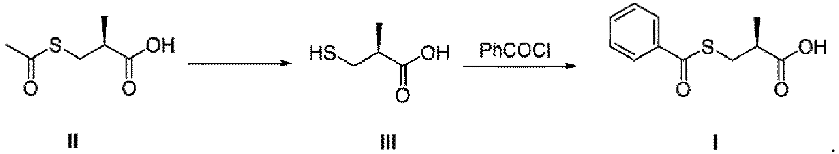

2. The method according to claim 1, wherein the hydrolyzing in step (a) is carried out in the presence of both an alkali and a metal.

3. The method according to claim 2, wherein the alkali is an inorganic alkali.

4. The method according to claim 3, wherein the inorganic alkali is sodium hydroxide or potassium hydroxide.

5. The method according to claim 2, wherein the metal is selected from the group consisting of copper powder, iron powder, zinc powder, aluminum powder, nickel powder, and cobalt powder, or any combination thereof; preferably zinc powder.

6. The method according to claim 2, wherein the mass of the metal is 0.5%-10%, preferably 2% with respect to the mass of S-(-)-3-acetylmercapto-2-methylpropanoic acid represented by formula II.

7. The method according to claim 1, wherein the reaction solvent in step (a) is an organic solvent, water, or a mixture of the organic solvent and water.

8. The method according to claim 7, wherein the organic solvent is selected from the group consisting of C₁-C₄ alcohols, C₆-C₈ alkanes, toluene, and ethyl acetate, or any combination thereof.

9. The method according to claim 1, wherein the ratio of the volume of the reaction solvent to the mass of S-(-)-3-acetylmercapto-2-methylpropanoic acid represented by formula II in step (a) is 1-10 mL/g, preferably 2 mL/g.

10. The method according to claim 1, wherein the molar ratio of benzoyl chloride to S-(-)-3-acetylmercapto-2-methylpropanoic acid represented by formula II in step (b) is 100%-120%.
